# EUROPÄISCHE PATENTSCHRIFT

(11) **EP 0 632 010 B1**
(45) Veröffentlichungstag und Bekanntmachung des Hinweises auf die Patenterteilung: **17.09.1997**
(21) Anmeldenummer: 94109700.8
(22) Anmeldetag: 23.06.1994
(51) Int. Cl.: C07C 67/00, C07C 69/34

(54) **Verfahren zur Herstellung von alpha,omega-Dicarbonsäurediestern**
Process for the preparation of diesters of alpha, omega dicarboxylic acids
Procédé de préparation de diesters d'acides alpha, oméga-dicarboxyliques

(30) Priorität: 01.07.1993 DE 4321842
(43) Veröffentlichungstag der Anmeldung: 04.01.1995
(73) Patentinhaber: BASF Aktiengesellschaft, 67063 Ludwigshafen (DE)
(72) Erfinder: Fischer, Rolf, Dr., D-69121 Heidelberg (DE)

(56) Entgegenhaltungen:
- DE-A- 1 668 730

## Beschreibung

Die vorliegende Erfindung betrifft ein Verfahren zur Herstellung von alpha,omega-Dicarbonsäurediestern durch Umsetzung von Cycloalkanonen mit Dimethylcarbonat in Gegenwart von Stickstoffbasen.

Aus der DE-A-16 68 730 ist bekannt, Cycloalkanone mit Dialkylcarbonaten in Gegenwart von Alkoholaten oder Alkalimetallen umzusetzen. Dabei werden nach Aufarbeitung mit wäßrigen Mineralsäuren alpha,omega- Dicarbonsäurediester isoliert, die ein Kohlenstoffatom mehr als die cyclischen Ausgangsketone enthalten. In den Beispielen 1 bis 6 ist die Umsetzung von Cyclodecanon bzw. Cyclododecanon zu Nonandicarbonsäurediestern bzw. Undecandicarbonsäurediestern beschrieben. Gearbeitet wird mit Alkalimetall- oder Alkoholatmengen von einem bis anderthalb Molen pro Mol eingesetztem Cycloalkanon in überschüssigem Dialkylcarbonat als Lösungsmittel.

Dieses Verfahren ist also durch einen hohen Mineralsalzanfall belastet.

Der vorliegenden Erfindung lag daher die Aufgabe zugrunde, den zuvor genannten Nachteilen abzuhelfen.

Demgemäß wurde ein neues und verbessertes Verfahren zur Herstellung von alpha,omega-Dicarbonsäurediestern der allgemeinen Formel I 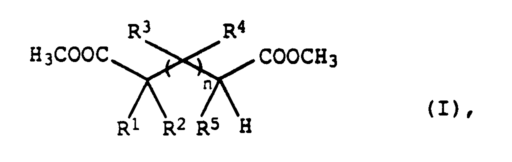 in der
- R¹,R²,R³,R⁴,R⁵: Wasserstoff, C₁- bis C₂₀-Alkyl, C₂- bis C₂₀-Alkenyl, C₂- bis C₂₀-Alkinyl, C₂- bis C₂₀-Alkoxycarbonyl, Nitro, C₁- bis C₂₀-Alkoxy und/oder Cyano und
- n: eine ganze Zahl von 1 bis 12
bedeutet, gefunden, welches dadurch gekennzeichnet ist, daß man Cycloalkanone der allgemeinen Formel II 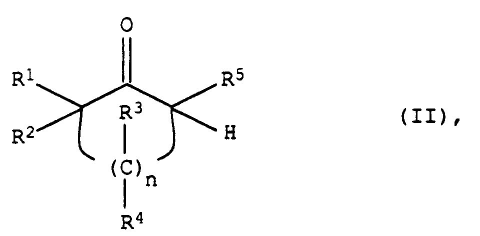 in der R¹, R², R³, R⁴, R⁵ und n die obengenannten Bedeutungen haben, mit Dimethylcarbonat in Gegenwart einer stickstoffhaltigen Base der allgemeinen Formel III 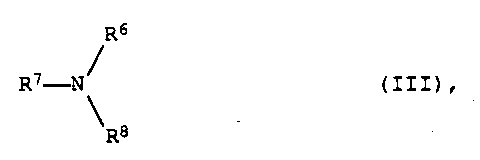 in der
- R⁶, R⁷, R⁸: Wasserstoff, C₁- bis C₂₀-Alkyl, C₃- bis C₈-Cycloalkyl oder C₇- bis C₂₀-Aralkyl oder R⁶ und R⁷ gemeinsam eine gegebenenfalls ein bis fünffach durch R¹ substituierte C₂- bis C₇-Alkylenkette
bedeuten, bei Temperaturen von 50 bis 300°C umsetzt.

Das erfindungsgemäße Verfahren läßt sich wie folgt durchführen:

Man kann die Cycloalkanone II mit Dimethylcarbonat in Gegenwart von stickstoffhaltigen Basen III z.B. in Druckapparaturen zusammengeben und bei Temperaturen von 50 bis 300°C, bevorzugt 100 bis 250°C, besonders bevorzugt 150 bis 230°C und Drücken von 0,01 bis 50 bar, bevorzugt 0,5 bis 5 bar, besonders bevorzugt bei dem sich im jeweiligen Reaktionsgemisch einstellenden Druck umsetzen.

Die Umsetzung kann in der Gasphase, bevorzugt aber in der Flüssigphase diskontinuierlich oder kontinuierlich durchgeführt werden.

Es kann vorteilhaft sein, die Umsetzung in Gegenwart von unter den Reaktionsbedingungen inerten Gasen wie Stickstoff oder Argon durchzuführen.

Die Umsetzung der Cycloalkanone II in der Flüssigphase kann beispielsweise so durchgeführt werden, daß man ein Gemisch aus II und gegebenenfalls einem Lösungsmittel in Gegenwart von Dimethylcarbonat und den stickstoffhaltigen Basen III auf die gewünschte Reaktionstemperatur erhitzt. Nach beendeter Reaktion kann das Reaktionsgemisch abgekühlt und zur Gewinnung der gewünschten alpha,omega-Dicarbonsäurediester fraktionierend destilliert werden.

Die erfindungsgemäße Umsetzung kann in Abwesenheit von Lösungsmitteln durchgeführt werden. Es kann jedoch vorteilhaft sein, in Anwesenheit von inerten Lösungsmitteln zu arbeiten. Als inerte Lösungsmittel können beispielsweise acyclische oder cyclische Ether wie Diethylether, Tetrahydrofuran und Dioxan, Aromaten wie Benzol, Toluol und die Xylole, chlorierte Kohlenwasserstoffe wie Chloroform und Methylenchlorid verwendet werden.

Die Menge an Lösungsmittel beträgt, bezogen auf die eingesetzten Cycloalkanone I, 0 bis 90 Gew.-%, bevorzugt 5 bis 80 Gew.-%, besonders bevorzugt 20 bis 60 Gew.-%.

Das Molverhältnis von Cycloalkanon II zum Dimethylcarbonat beträgt in der Regel 10 : 1 bis 1 : 1, bevorzugt 5 : 1 bis 2 : 1. Es ist auch möglich, in überschüssigem Dimethylcarbonat als Lösungsmittel zu arbeiten.

Als stickstoffhaltige Basen III eignen sich Ammoniak, primäre, sekundäre und tertiäre Amine mit aliphatischen, cycloaliphatischen, heteroaromatischen, und/oder araliphatischen Substituenten oder Gemische derartiger stickstoffhaltiger Basen. Dabei können zwei aliphatische Substituenten auch zu einem Ring geschlossen sein. Geeignet sind auch Diamine.

Beispiele hierfür sind:
- Ammoniak,
- Methylamin, Ethylamin, Hexylamin und Cyclohexylamin,
- Dimethylamin, Diethylamin, Dibutylamin und Dicyclohexylamin,
- Trimethylamin, Dimethylethylamin, Triethylamin, Tri-n-propylamin, Tri-iso-propylamin, Tributylamin, Trioctylamin, Tricyclohexylamin, Trihexadecylamin, Tricyclohexylamin, Diphenylmethylamin, Dimethylbenzlamin, Dibenzylmethylamin, Tribenzylamin, N,N-Tetramethylhexamethylendiamin, Hexamethylendiamin und Tetramethylendiamin,
- 4-Dimethylaminpyridin, Urotropin, Piperidin, N-Methylpiperidin, Pyrrolidin, N-Methylpyrrolidin, Hexamethylenimin, N-Ethylhexamethylenimin, N-Methylimidazol, 1,4-Diazabicyclo[4,3,0]octan (DABCO), Morpholin, Piperazin und Pyrrolidin.

Weiterhin eignen sich Amidine wie 1,5-Diazabicyclo[4,3,0]non-5-en (DBN), 1,8-Diazabicyclo-[5,4,0]undec-7-en (DBU) und Guanidin. Bevorzugt sind die tertiären Amine, besonders bevorzugt C₁- bis C₈-Trialkylamine.

Das Molverhältnis von Cycloalkanon II zu den stickstoffhaltigen Basen beträgt in der Regel 100 : 1 bis 1 : 1 , bevorzugt 20 : 1 bis 3 : 1.

Die als Ausgangsverbindungen verwendeten Cycloalkanone II sind allgemein zugängliche Verbindungen, die z. B. nach Houben-Weyl, Methoden der Organischen Chemie, 4. Auflage, Band VII/2a, Seiten 637 bis 641 und 699 bis 711 (1973) herstellbar sind.

Beispiele für derartige Cycloalkanone II sind Cyclopentanon, Cylcohexanon, Cylcoheptanon, Cyclooctanon, Cyclododecanon, 2-Methylcyclopentanon, 2-Ethylcyclohexanon, 2,5-Dimethylcyclopentanon, 2-Cyanocyclohexanon, 2-Nitrocyclohexanon, 2-Nitrocyclopentanon, 2-Methylcyclooctanon, 2-Methoxycarbonylcyclohexanon, 2-Vinylcyclohexanon, 2,6-Dimethylcyclohexanon, 2-Methoxycyclohexanon.

Die Substituenten R¹, R², R³, R⁴, R⁵, R⁶, R⁷, R⁸ und der Index n in den Verbindungen I, II und III haben folgende Bedeutungen:
R¹, R², R³, R⁴, R⁵, R⁶, R⁷, R⁸
   - Wasserstoff,
   - C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
R¹, R², R³, R⁴, R⁵
   - C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl, 2,2-Dimethyl-pent-1-en-1-yl, ...,
   - C₂- bis C₂₀-Alkinyl, bevorzugt C₂- bis C₈-Alkinyl, besonders bevorzugt C₂- bis C₄-Alkinyl wie Ethinyl, 1-Propinyl, 2-Propinyl, 1-Butinyl, 2-Butinyl, 3-Butinyl, 1-Methyl-2-propinyl,
   - C₂- bis C₂₀-Alkoxycarbonyl, bevorzugt C₂- bis C₉-Alkoxycarbonyl wie Methoxycarbonyl, Ethoxycarbonyl, n-Propoxycarbonyl, iso-Propoxycarbonyl, n-Butoxycarbonyl, iso-Butoxycarbonyl, sec.-Butoxycarbonyl, tert.-Butoxycarbonyl, n-Pentoxycarbonyl, iso-Pentoxycarbonyl, sec.-Pentoxycarbonyl, neo-Pentoxycarbonyl, 1,2-Dimethylpropoxycarbonyl, n-Hexoxycarbonyl, iso-Hexoxycarbonyl, sec.-Hexoxycarbonyl, n-Heptoxycarbonyl, iso-Heptoxycarbonyl, n-Octoxycarbonyl und iso-Octoxycarbonyl, besonders bevorzugt C₁- bis C₅-Alkoxycarbonyl wie Methoxycarbonyl, Ethoxycarbonyl, n-Propoxycarbonyl, iso-Propoxycarbonyl, n-Butoxycarbonyl, iso-Butoxycarbonyl, sec.-Butoxycarbonyl, tert.-Butoxycarbonyl,
   - Nitro,
   - C₁- bis C₂₀-Alkoxy, bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy und iso-Octoxy, besonders bevorzugt C₁- bis C₄-Alkoxy wie Methoxy, Ethoxy, n-Propoxy, iso-Propoxy, n-Butoxy, iso-Butoxy, sec.-Butoxy, tert.-Butoxy,
   - Cyano und
R⁶, R⁷, R⁸
   - C₃ bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
   - C₇ bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl oder
R⁶ und R⁷ gemeinsam
   - eine gegebenenfalls ein bis fünffach durch R¹ substituierte C₂- bis C₇-Alkylenkette wie Ethylen, Propylen, Butylen und Pentylen, bevorzugt Butylen und Pentylen,
n
   - eine ganze Zahl von 1 bis 12, bevorzugt eine ganze Zahl von 2 bis 9, besonders bevorzugt eine ganze Zahl von 2 bis 5, insbesondere 2 oder 3.

### Beispiele

### Beispiel 1

Ein Gemisch aus 22,4 g 2,5-Dimethylcyclopentanon, 45 g Dimethylcarbonat und 3 g Ethyldimethylamin wurde in einem Autoklaven auf 200°C aufgeheizt und 5 Stunden bei dieser Temperatur gerührt. Nach dem Abkühlen des Autoklaven wurde der flüssige Reaktionsaustrag fraktionierend destilliert. Dabei wurden 7,4 g unumgesetztes 2,5-Dimethylcyclopentanon (33 %, bezogen auf eingesetztes 2,5-Dimethylcyclopentanon) und 17,4 g 2,5-Dimethyladipinsäuredimethylester (Ausbeute 43 %, bezogen auf eingesetztes 2,5-Dimethylcyclopentanon, Selektivität 64 %) erhalten.

### Beispiel 2

Ein Gemisch aus 24,5 g Cyclohexanon, 115 g Dimethylcarbonat und 3,7 g Ethyldimethylamin wurde wie in Beispiel 1 beschrieben umgesetzt. Gaschromatographisch (GC Fl.-%) wurde festgestellt, daß der Reaktionsaustrag (ohne unumgesetztes Dimethylcarbonat und ohne gebildetes Methanol) zu 19 % aus nicht umgesetztem Cyclohexanon, zu 35 % aus Pimelinsäuredimethylester und zu 10 % aus 2-Methylpimelinsäuredimethylester bestand.

### Beispiel 3

Wurde in Beispiel 2 anstelle von Cyclohexanon als Ausgangsprodukt Cyclohexanon-2-carbonsäureethylester eingesetzt, so wurde als Hauptprodukt ein Gemisch aus Pimelinsäuredimethylester und Pimelinsäuremethylethylester identifiziert.

### Beispiel 4

Ein Gemisch aus 9,8 g 2-Methylcyclopentanon, 45,9 g Dimethylcarbonat und 1,20 g Triethylamin wurde wie in Beispiel 1 beschrieben umgesetzt. Gaschromatographisch wurde festgestellt (GC Fl.-%), daß der Reaktionsaustrag neben unumgesetztem Keton, Dimethylcarbonat und gebildetem Methanol als Hauptprodukte 2-Methyl- und 2,5-Dimethyladipinsäuredimethylester im Molverhältnis 1 : 1,3 enthielt.

## Patentansprüche

1. Verfahren zur Herstellung von alpha,omega-Dicarbonsäurediestern der allgemeinen Formel I 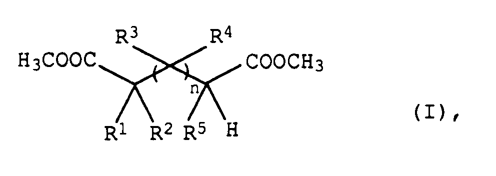 in der
R¹,R²,R³,R⁴,R⁵ Wasserstoff, C₁- bis C₂₀-Alkyl, C₂- bis C₂₀-Alkenyl, C₂- bis C₂₀-Alkinyl, C₂- bis C₂₀-Alkoxycarbonyl, Nitro, C₁- bis C₂₀-Alkoxy und/oder Cyano und
n eine ganze Zahl von 1 bis 12
bedeutet, dadurch gekennzeichnet, daß man Cycloalkanone der allgemeinen Formel II 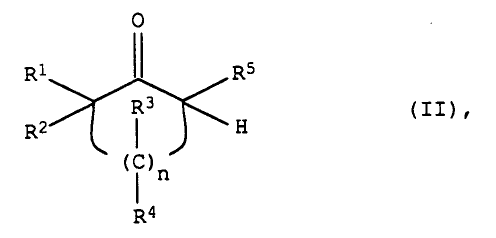 in der R¹, R², R³, R⁴, R⁵ und n die obengenannten Bedeutungen haben, mit Dimethylcarbonat in Gegenwart einer stickstoffhaltigen Base der allgemeinen Formel III 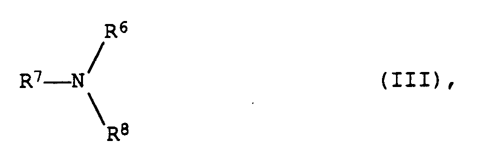 in der
R⁶, R⁷, R⁸ Wasserstoff, C₁- bis C₂₀-Alkyl, C₃- bis C₈-Cycloalkyl oder C₇- bis C₂₀-Aralkyl oder R⁶ und R⁷ gemeinsam eine gegebenenfalls ein bis fünffach durch R¹ substituierte C₂- bis C₇-Alkylenkette
bedeuten, bei Temperaturen von 50 bis 300°C umsetzt.

2. Verfahren zur Herstellung von alpha,omega-Dicarbonsäurediestern I nach Anspruch 1, dadurch gekennzeichnet, daß man als stickstoffhaltige Base 1,5-Diazabicyclo-[4,3,0]non-5-en und 1,8-Diazabicyclo[5,4,0]undec-7-en einsetzt.

3. Verfahren zur Herstellung von alpha,omega-Dicarbonsäurediestern I nach Anspruch 1, dadurch gekennzeichnet, daß man die Cycloalkanone II zu den stickstoffhaltigen Basen im Molverhältnis von 100 : 1 bis 1 : 1 einsetzt.

4. Verfahren zur Herstellung von alpha,omega-Dicarbonsäurediestern I nach Anspruch 1, dadurch gekennzeichnet, daß man die Umsetzung bei Temperaturen von 100 bis 250°C durchführt.

5. Verfahren zur Herstellung von alpha,omega-Dicarbonsäurediestern I nach Anspruch 1, dadurch gekennzeichnet, daß man die Umsetzung bei Temperaturen von 150 bis 230°C durchführt.

6. Verfahren zur Herstellung von alpha,omega-Dicarbonsäurediestern I nach Anspruch 1, dadurch gekennzeichnet, daß man die Umsetzung bei Drücken von 0,01 bis 50 bar durchführt.

7. Verfahren zur Herstellung von alpha,omega-Dicarbonsäurediestern I nach Anspruch 1, dadurch gekennzeichnet, daß man die Umsetzung bei dem sich einstellenden Druck im Druckreaktor durchführt.

## Claims

1. A process for preparing alpha,omega-dicarboxylic diesters of the general formula I 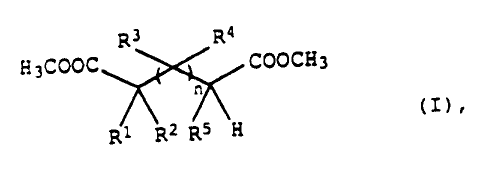 where
R¹, R², R³, R⁴, R⁵ are hydrogen, C₁-C₂₀-alkyl, C₂-C₂₀-alkenyl, C₂-C₂₀-alkynyl, C₂-C₂₀-alkoxycarbonyl, nitro, C₁-C₂₀-alkoxy and/or cyano and
n is an integer from 1 to 12,
which comprises reacting cycloalkanones of the general formula II 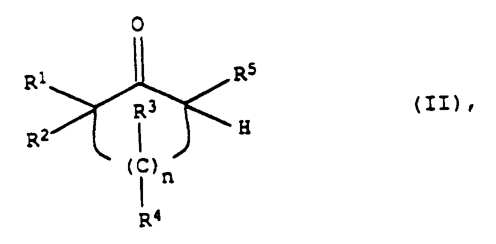 where R¹, R², R³, R⁴, R⁵ and n have the abovementioned meanings, with dimethyl carbonate in the presence of a nitrogenous base of the general formula III 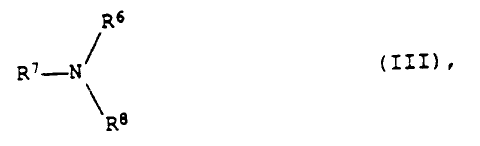 where
R⁶, R⁷, R⁸ are hydrogen, C₁-C₂₀-alkyl, C₃-C₈-cycloalkyl or C₇-C₂₀-aralkyl or R⁶ and R⁷ are together a C₂-C₇-alkylene chain which is unsubstituted or substituted one to five times by R¹,
at from 50 to 300°C.

2. A process for preparing alpha,omega-dicarboxylic diesters I as claimed in claim 1, wherein 1,5-diazabicyclo[4,3,0]non-5-ene and 1,8-diazabicyclo[5,4,0]undec-7-ene are employed as nitrogenous base.

3. A process for preparing alpha,omega-dicarboxylic diesters I as claimed in claim 1, wherein the molar ratio employed of cycloalkanones II to nitrogenous bases is from 100:1 to 1:1.

4. A process for preparing alpha,omega-dicarboxylic diesters I as claimed in claim 1, wherein the reaction is carried out at from 100 to 250°C.

5. A process for preparing alpha,omega-dicarboxylic diesters I as claimed in claim 1, wherein the reaction is carried out at from 150 to 230°C.

6. A process for preparing alpha,omega-dicarboxylic diesters I as claimed in claim 1, wherein the reaction is carried out under 0.01 to 50 bar.

7. A process for preparing alpha,omega-dicarboxylic diesters I as claimed in claim 1, wherein the reaction is carried out under the pressure which is set up in the pressure reactor.

## Revendications

1. Procédé de préparation de diesters d'acides α,ω-dicarboxyliques de formule générale I 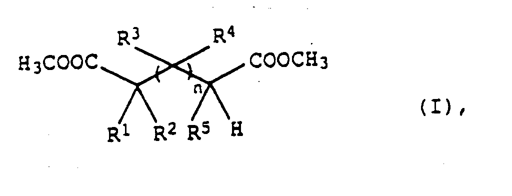 dans laquelle
R¹, R², R³, R⁴, R⁵ représentent des atomes d'hydrogène ou des restes alkyle en C₁-C₂₀, alcényle en C₂-C₂₀, alcynyle en C₂-C₂₀, alcoxycarbonyle en C₂-_{C20}, nitro, alcoxy en C₁-C₂₀ et/ou cyano et
n est un nombre entier de 1 à 12,
caractérisé en ce que l'an fait réagir, à des températures de 50 à 300°C, des cycloalcanones de formule générale II 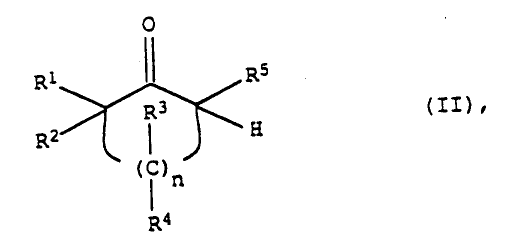 dans laquelle R¹, R², R³, R⁴, R⁵ et n ont les significations données ci-dessus, avec du carbonate de diméthyle en présence d'une base azotée de formule générale III 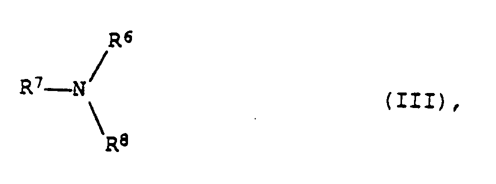 dans laquelle
R⁶, R⁷, R⁸ représentent des atomes d'hydrogène au des restes alkyle en C₁-C₂₀, cyclaalkyle en C₃-C₈ au aralkyle en C₇-C₂₀, au R⁶ et R⁷ représentent ensemble une chaîne alkylène en C₂-C₇ éventuellement substituée une à cinq fois par R¹.

2. Procédé de préparation de diesters d'acides α,ω-dicarboxyliques selon la revendication 1, caractérisé en ce que l'an utilise, comme base azotée, du 1,5-diazabicyclo[4.3.0]non-5-ène et du 1,8-diazabicyclo[5.4.0]undéc-7-ène.

3. Procédé de préparation de diesters d'acides α,ω-dicarboxyliques selon la revendication 1, caractérisé en ce que l'on utilise les cycloalcanones II dans un rapport malaire aux bases azotées de 100:1 à 1:1.

4. Procédé de préparation de diesters d'acides α,ω-dicarboxyliques selon la revendication 1, caractérisé en ce que l'on conduit la réaction à des températures de 100 à 250°C.

5. Procédé de préparation de diesters d'acides α,ω-dicarboxyliques selon la revendication 1, caractérisé en ce que l'on conduit la réaction à des températures de 150 à 230°C.

6. Procédé de préparation de diesters d'acides α,ω-dicarboxyliques selon la revendication 1, caractérise en ce que l'on conduit la réaction sous des pressions de 0,01 à 50 bar.

7. Procédé de préparation de diesters d'acides α,ω-dicarboxyliques selon la revendication 1, caractérisé en ce que l'on conduit la réaction sous la pression qui s'établit dans le réacteur résistant à la pression.
